# EUROPEAN PATENT SPECIFICATION

(11) **EP 1 414 816 B1**
(45) Date of publication and mention of the grant of the patent: **09.02.2005**
(21) Application number: 02767277.3
(22) Date of filing: 30.07.2002
(51) Int. Cl.: C07D 403/06

(54) **STABLE POLYMORPH OF FLIBANSERIN, TECHNICAL PROCESS FOR ITS PREPARATION AND THE USE THEREOF FOR PREPARING MEDICAMENTS**
STABILES POLYMORPH VON FLIBANSERIN, INDUSTRIELLES VERFAHREN ZU DESSEN HERSTELLUNG UND DESSEN VERWENDUNG ZUR HERSTELLUNG VON MEDIKAMENTEN
POLYMORPHE STABLE DE FLIBANSERINE, PROCEDE DE PREPARATION ASSOCIE ET UTILISATION DUDIT POLYMORPHE DANS LA PREPARATION DE MEDICAMENTS

(30) Priority: 02.08.2001 EP 01118593; 19.12.2001 EP 01130180
(43) Date of publication of application: 06.05.2004
(60) Divisional application: 04026410.3
(73) Proprietor: BIDACHEM S.p.A., I-24040 Fornovo San Giovanni (Bergamo) (IT)
(72) Inventor: BOMBARDA, Carlo, CHESTER, VA 23836 (US); DUBINI, Enrica, I-ZIP 20135 MILANO (IT); EZHAYA, Antoine, I-ZIP 20141 MILANO (IT)
(74) Representative: Hammann, Heinz, Dr.
(86) International application number: PCT/EP2002/008466
(87) International publication number: WO 2003/014079

(56) References cited:
- EP-A- 0 526 434
- BORSINI F ET AL: "BIMT 17, A 5-HT2A RECEPTOR ANTAGONIST AND 5-HT1A RECEPTOR FULL AGONIST IN RAT CEREBRAL CORTEX" NAUNYN-SCHMIEDEBERG'S ARCHIVES OF PHARMACOLOGY, SPRINGER, BERLIN, DE, vol. 352, no. 3, September 1995 (1995-09), pages 276-282, XP001057819 ISSN: 0028-1298
- GIRON ET AL: "Thermal analysis and calorimetric methods in the characterisation of polymorphs and solvates" THERMOCHIMICA ACTA, ELSEVIER SCIENCE PUBLISHERS, AMSTERDAM, NL, vol. 248, 1995, pages 1-59, XP002101808 ISSN: 0040-6031
- BERNSTEIN, J. ET AL.: "Concomitant Polymorphs" ANGEWANDTE CHEMIE, INT. ED., 1999, pages 3441-3461, XP002219563

## Description

The invention relates to the polymorph A of flibanserin, to a technical process for the preparation thereof, as well as to the use thereof for preparing medicaments.

### Background of the invention

The compound 1-[2-(4-(3-trifluoromethyl-phenyl)piperazin-1-yl)ethyl]-2,3-dihydro-1H-benzimidazol-2-one (flibanserin) is disclosed in form of its hydrochlorid in European Patent Application EP-A-526434 and has the following chemical structure:

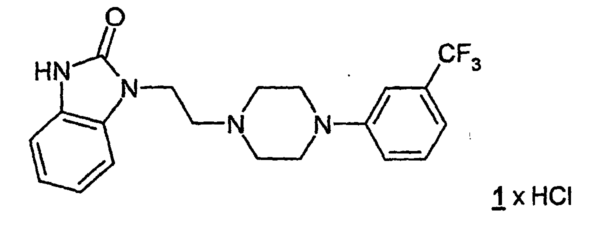

Flibanserin shows affinity for the 5-HT_{1A} and 5-HT₂-receptor. It is therefore a promising therapeutic agent for the treatment of a variety of diseases, for instance depression, schizophrenia, Parkinson, anxiety, sleep disturbances, sexual and mental disorders and age associated memory impairment.

A certain phamaceutical activity is of course the basic prerequisite to be fulfilled by a pharmaceutically active agent before same is approved as a medicament on the market. However, there are a variety of additional requirements a pharmaceutically active agent has to comply with. These requirements are based on various parameters which are connected with the_nature of the active substance itself. Without being restrictive, examples of these parameters are the stability of the active agent under various environmental conditions, its stability during production of the pharmaceutical formulation and the stability of the active agent in the final medicament compositions. The pharmaceutically active substance used for preparing the pharmaceutical compositions should be as pure as possible and its stability in long-term storage must be guaranteed under various environmental conditions. This is absolutely essential to prevent the use of pharmaceutical compositions which contain, in addition to the actual active substance, breakdown products thereof, for example. In such cases the content of active substance in the medicament might be less than that specified.

Uniform distribution of the medicament in the formulation is a critical factor, particularly when the medicament has to be given in low doses. To ensure uniform distribution, the particle size of the active substance can be reduced to a suitable level, e.g. by grinding. Since breakdown of the pharmaceutically active substance as a side effect of the grinding (or micronising) has to be avoided as far as possible, in spite of the hard conditions required during the process, it is absolutely essential that the active substance should be highly stable throughout the grinding process. Only if the active substance is sufficiently stable during the grinding process is it possible to produce a homogeneous pharmaceutical formulation which always contains the specified amount of active substance in reproducible manner.

Another problem which may arise in the grinding process for preparing the desired pharmaceutical formulation is the input of energy caused by this process and the stress on the surface of the crystals. This may in certain circumstances lead to polymorphous changes, to a change in the amorphous configuration or to a change in the crystal lattice. Since the pharmaceutical quality of a pharmaceutical formulation requires that the active substance should always have the same crystalline morphology, the stability and properties of the crystalline active substance are subject to stringent requirements from this point of view as well.

The stability of a pharmaceutically active substance is also important in pharmaceutical compositions for determining the shelf life of the particular medicament; the shelf life is the length of time during which the medicament can be administered without any risk. High stability of a medicament in the abovementioned pharmaceutical compositions under various storage conditions is therefore an additional advantage for both the patient and the manufacturer.

Apart from the requirements indicated above, it should be generally borne in mind that any change to the solid state of a pharmaceutical composition which is capable of improving its physical and chemical stability gives a significant advantage over less stable forms of the same medicament.

The aim of the invention is thus to provide a new, stable crystalline form of the compound flibanserin which meets the stringent requirements imposed on pharmaceutically active substances as mentioned above.

### Detailed description of the invention

Surprisingly, it has been found that the free base of flibanserin in a specific polymophic form fulfils the requirements mentioned hereinbefore.

Moreover it has been found that, depending on the choice of conditions which can be applied during the synthesis of flibanserin the free base occurs in different crystalline modifications, polymorphs A and B.

It has been found that these different modifications can be deliberately produced by a suitable choice of the process conditions used in the manufacturing process.

Surprisingly, it has been found that polymorph A, which can be obtained in crystalline form by choosing specific reaction conditions, meets the stringent requirements mentioned above and thus solves the problem on which the present invention is based. Accordingly the present invention relates to polymorph A of flibanserin. Polymorph A of flibanserin is characterised by a melting point of about 161 °C (determined via DSC; heating rate 10 K/min).

Polymorph B, the less stable modification of flibanserin displays a melting point of about 120°C (determined via DSC; heating rate 10 K/min). Whereas polymorph B shows little stability under the effects of for instance mechanical stress produced by grinding, polymorph A turned out to fulfill the aforementioned stability requirements.

According to another aspect, the present invention relates to a process for the manufacture of polymorph A of flibanserin in technical scale. The process according to the inventon is illustrated in diagram 1.

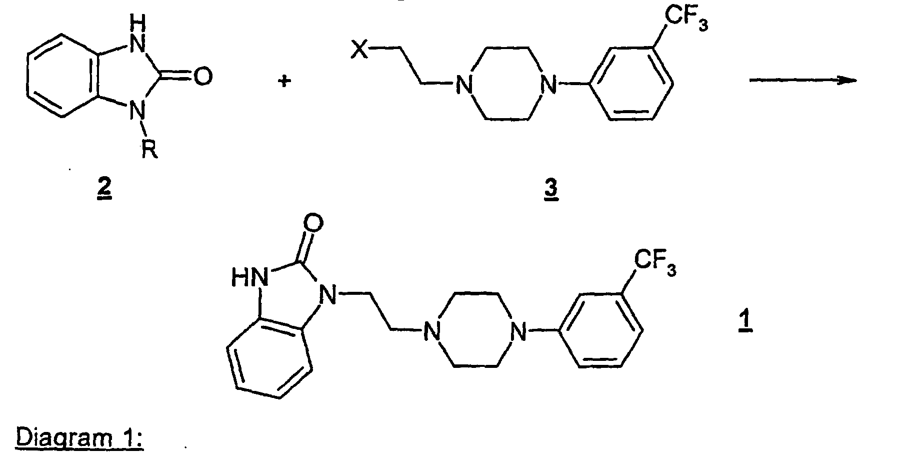

The benzimidazolone **2** is reacted with the piperazine-derivative **3** under basic reaction conditions in a suitable solvent to lead to **1**. In **2** the group R denotes an amino protecting group. The protecting group used may be any of the groups commonly used to protect the amino function. Examples include groups selected from alkyl, substituted alkyl, heterosubstituted alkyl, unsaturated alkyl, alkyl substituted heteroatoms, substituted or unsubstituted phenyl, substituted or unsubstituted benzyl, alkyloxycarbonyl groups and aryloxycarbonyl groups. Preferred protecting groups are selected from butyl, 1,1-diphenylmethyl, methoxymethyl, benzyloxymethyl, trichloroethoxymethyl, pyrrolidinomethyl, cyanomethyl, pivaloyloxymethyl, allyl, 2-propenyl, t-butyldimethylsilyl, methoxy, thiomethyl, 4-methoxyphenyl, benzyl, 4-methoxybenzyl, 2,4-dimethoxybenzyl, 2-nitrobenzyl, t-butoxycarbonyl, benzyloxycarbonyl, phenoxy carbonyl, 4-chloro-phenoxycarbonyl, 4-nitro-phenoxycarbonyl, methoxycarbonyl and ethoxycarbonyl. Among them the preferred protecting groups are selected from t-butoxycarbonyl, ethoxycarbonyl, methoxycarbonyl, benzyloxycarbonyl, phenoxycarbonyl and 2-propenyl, the latter being most preferred. X in **3** represents a leaving group selected from chlorine, bromine, iodine, methanesulphonate, trifluoromethanesulphonate or para-toluenesulphonate. Preferably X denotes chlorine, bromine or iodine, chlorine being most preferred. Suitable solvents are selected from water, alcohols and mixtures of water with alcohols, polar aprotic solvents and mixtures thereof with water. Preferred solvents are selected from the group consisting of dimethylformamid, dimethylsulfoxid, acetonitrile, tetrahydrofurane, dioxane, methanol, ethanol isopropanaol and mixtures of one or several of the aformenetioned solvents with water. Preferred solvents are those being readily miscible with water. Preferably, a mixture of water with one of the alcohols methanol, ethanol or isopropanol is used as the solvent. In a preferred embodiment a mixture of water and isopropanol is used as the solvent. The base used may be an alkali metal- or alkaline earth metal carbonate of lithium, sodium, potassium, calcium such as sodium carbonate, lithium carbonate, potassium carbonate, calcium carbonate and preferably potassium carbonate. It is also possible to use the hydrogen carbonates of lithium, sodium and potassium. Preferably, the alkali metal- or alkaline earth metal hydroxides of lithium, sodium, potassium, magnesium, calcium, but preferably sodium hydroxide, potassium hydroxide, lithium hydroxide and calcium hydroxide in alcohols or water may also be used. Most preferred base is sodium hydroxide. The base is preferably added in form of its aqueous solution, preferably in form of concentrated aqueous solutions, for example in concentrations between 30 - 50% weight/volume. In a preferred embodiment aqueous sodium hydroxide solution in a concentration of about 45% weight/volume is used.

The compounds **2** and **3** are introduced into the reaction in a molar ratio of between 1:1 to 1:2, preferably in a molar ratio of between 1:1.1 to 1:1.5.

As mentioned hereinbefore a mixture of water and isopropanol is used as a preferred solvent mixture for the conduction of the process according to the invention. In this solvent mixture the weight-ratio of water to isopropanol in the preferred solvent mixture is between 10:1 and 1:1, more preferred between 8:1 and 3:1, particular preferred between 7:1 and 5:1. Per mol of compound **2** about 2 - 10 kg, preferably 3 - 8 kg, more preferred 4 - 7 kg of the aforementioned solvent mixture are used. In a preferred embodiement the reaction is conducted using aqueous sodium hydroxide solution in a concentration of about 45% weight/volume as the base. Per mol of **2** about 0.1 - 1.5 kg, preferably 0.2 - 1.0 kg, particularly preferred 0.3 - 0.6 kg of the aforementioned sodium hydroxide solution are used. The reaction mixture containing **2, 3** and the base in the aforementioned suitable solvent is preferably heated to at least 50°C. In a preferred embodiment the reaction temperature is in a range of between 60°C to the boiling point of the solvent. Particularly preferred is a temperature between 70 - 90°C. The reaction mixture is heated at the aformentioned temperature for about 10 minutes to about 12 hours, preferably for about 15 minutes to about 6 hours, more preferably for about 30 minutes to about 3 hours. The reaction mixture is preferably heated at the aformentioned temperature for about 45 to 60 minutes.

Subsequently the protective group R is cleaved. The cleaving conditions depend on the choice of group R. If R denotes for instance benzyl, cleavage is conducted via hydrogenation in acetic acid in the presence of an appropriate catalyst (e.g. Pd on charcoal) or it can be cleaved in aqueous HBr. In case R is methoxycarbonyl, ethoxycarbonyl, phenoxy carbonyl, 4-nitrophenoxycarbonyl it can be cleaved for example by using aqueous alkaline solutions such as NaOH (aq) or KOH(aq). In case R is t-butoxycarbonyl it can be cleaved for instance in aqueous HCl or HBr. In case R denotes 2-propenyl, the particularly preferred protective group according to the invention, cleavage of R is effected via acidic reaction conditions. In a particularly preferred process according to the invention the 2-propenyl group is cleaved by using a strong mineral acid, preferably an acid selected from the group consisting of hydrobromic acid, hydrochloric acid and sulfuric acid, more preferably hydrochloric acid. Hydrochloric acid can be added in gaseous form or in form of its aqueous solutions, the addition of aqueous solutions being preferred. Particularly preferred is the addition of hydrochloric acid in form of its concentrated solution (about 36% weight/volume). Per mol **2** at least one mol of hydrochloric acid is to be added. Preferably the amount of added concentrated hydrochloric acid (36% weight/volume) per mol **2** is between 50 - 500g, more preferred between 80 - 250g. Particularly preferred about 120 - 160 g of concentrated (36% w/v) aqueous hydrochloric acid are added per mol **2** used. Additional water can be optionally added. At a temperature of about 70 - 90°C about 30 - 70%, preferably about 35 - 60% of the solvent is removed via distillation. At a temperature of about 60 - 80°C the pH of the remaining residue is adjusted to about 5 - 9, preferably to about 6 - 8 by addition of aqueous sodium hydroxide (45% w/v). At a temperature of about 40 - 55°C the pH is adjusted to about 8 - 9 by addition of aqueous sodium hydroxide (45% w/v). Subsequently the mixture is cooled to about 20-40°C, preferably about 30-35°C and centrifuged. The residue thus obtained is washed with about 100 to 750 ml water per mol introduced **2**, preferably with about 200 to 500, particularily preffered with about 300 to 400 ml water per mol introduced **2** and isopropanol (about 50 to 250 g per mol **2**, preferably about 100 to 200 g per mol **2**) and then with water until chlorides elimination. Optionally the product thus obtained can be subjected to another purification step. Preferably, said purification is conducted via crystallization of **1** from fror instance acetone.

One aspect of the present invention relates to flibanserin polymorph A obtainable via the method described above.

The following example of synthesis serves to illustrate a method of preparing polymorph A of flibanserin. It is to be regarded only as a possible method described by way of example, without restricting the invention to its contents.

### Example:

375 kg of 1-[(3-trifluoromethyl)phenyl]-4-(2-cloroethyl)piperazin are charged in a reactor with 2500 kg of water and 200 kg of aqueous Sodium Hydroxide 45%. Under stirring 169.2 kg of 1-(2-propenyl)-1,3-dihydro-benzimidazol-2H-one, 780 kg of isopropanol, 2000 kg of water and 220 kg of aqueous Sodium Hydroxide 45% are added. The reaction mixture is heated to 75-85°C and 160 kg of concentrated hydrochloric acid and 200 kg of water are added. The reaction mixture is stirred at constant temperature for about 45 minutes. After distillation of a mixture of water and Isopropanol (about 3000 kg) the remaining residue is cooled to about 65-75°C and the pH is adjusted to 6.5 - 7.5 by addition of 125 kg of aqueous Sodium Hydroxide 45%. After cooling to a temperature of 45-50°C, the pH value is adjusted to 8-9 by addition of about 4 kg of aqueous Sodium Hydroxide 45%. Subsequently the mixture is cooled to 30-35°C and centrifuged. The residue thus obtained is washed with 340 I of water and 126 I of isopropanol and then with water until chlorides elimination. The wet product is dried under vacuum at a temperature of about 45-55°C which leads to 358 kg of crude flibanserin polymorph A. The crude product thus obtained is loaded in a reactor with 1750 kg of Acetone and the resulting mixture is heated under stirring until reflux. The obtained solution is filtered and the filtrate is concentrated by distillation. The temperature is maintained for about 1 hour 0-5°C, then the precipitate solid is isolated by filtration and dried at 55°C for at least 12 hours. The final yield is 280 kg of pure flibanserin polymorph A.

As mentioned hereinbefore flibanserin polymorph A was characterised by DSC (Differantial Scanning Calorimetry). The peak temperature determined for polymorph A is about 161°C. For the characterization via DSC a Mettler TA 3000 System equipped with TC 10-A processor and DSC 20 cell was applied. The heating rate was 10 K/min.

The flibanserin polymorph A was additionally characterised by powder x-ray diffractometry. The x-ray powder diffraction pattern for polymorph A was obtained according to the following conditions:

| | | |
|---|---|---|
| Equipment | Philips PW 1800/10 diffractometer equipped with a digital microvax 2000. | |
| | | |
| Setting parameters | X-ray | |
| | Type tube | Cu (long fine focus) |
| | Wavelenghts (λ) | K_{α1} = 1.54060 A K_{α2} = 1.54439 Å |
| | Intensity ratio (α2/ α1) | 0.500 |
| | | |
| | Start angle [°2Θ] | 2.000 |
| | End angle [°2Θ] | 60.000 |
| | Step size [°2Θ] | 0.020 |
| | Maximum intensity[s] | 7310.250 |
| | | |
| | Type of scan | continuous |
| | | |
| | Minimum peak tip width | 0.00 |
| | Maximum peak tip width | 1.00 |
| | Peak base width | 2.00 |
| | Minimum significance | 0.75 |
| | Number of peaks | 69 |
| | | |
| Generator | high voltage | 50 KV |
| | tube current | 30 mA |

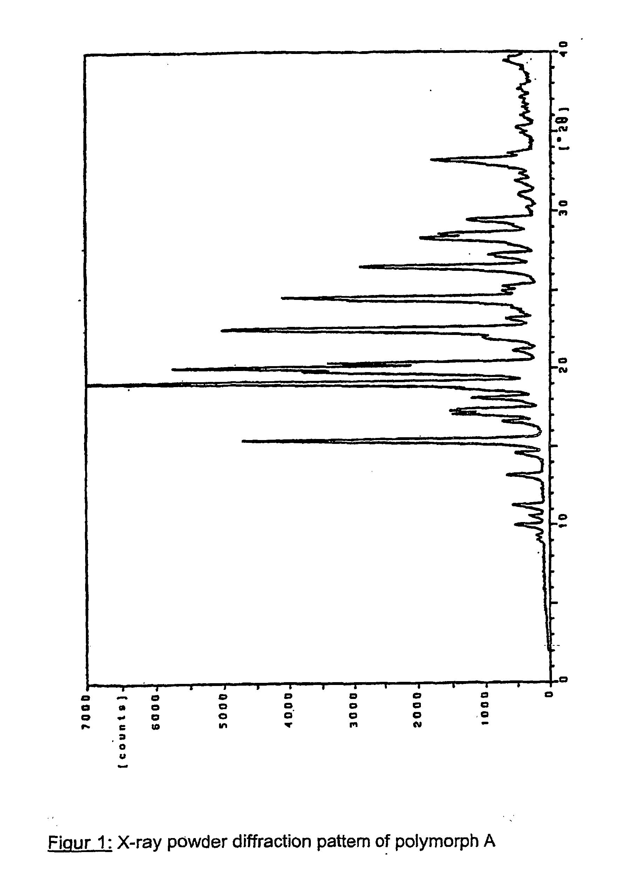

The powder x-ray diffraction pattern obtained for polymorph A is illustrated in figure 1. The appropiate values are collected in table 1.

**Table 1:**

| Angle [°2Θ] | d-value α1 [Å] | d-value α 2 [Å] | Peak width [°2Θ] | Peak int [counts] | Back. int [counts] | Rel. int [%] | Signif. |
|---|---|---|---|---|---|---|---|
| 5.195 | 16.9967 | 17.0390 | 0.960 | 8 | 69 | 0.1 | 1.05 |
| 9.045 | 9.7689 | 9.7931 | 0.100 | 92 | 96 | 1.3 | 0.97 |
| 9.335 | 9.4660 | 9.4896 | 0.080 | 114 | 98 | 1.6 | 0.88 |
| 10.025 | 8.8160 | 8.8379 | 0.140 | 400 | 100 | 5.5 | 7.18 |
| 10.595 | 8.3430 | 8.3637 | 0.140 | 204 | 102 | 2.8 | 3.46 |
| 11.290 | 7.8309 | 7.8503 | 0.140 | 467 | 104 | 6.4 | 6.91 |
| 13.225 | 6.6891 | 6.7058 | 0.180 | 548 | 112 | 7.5 | 13.10 |
| 14.595 | 6.0642 | 6.0793 | 0.180 | 404 | 121 | 5.5 | 9.17 |
| 15.460 | 5.7268 | 5.7410 | 0,140 | 4186 | 125 | 57.3 | 23.20 |
| 16.655 | 5.3185 | 5.3317 | 0.200 | 515 | 130 | 7.0 | 12.38 |
| 17.085 | 5.1856 | 5.1985 | 0.100 | 1347 | 132 | 18.4 | 2.78 |
| 17.285 | 5.1260 | 5.1388 | 0.060 | 1399 | 135 | 19.1 | 2.26 |
| 17.420 | 5.0866 | 5.0992 | 0.100 | 1204 | 135 | 16.5 | 4.71 |
| 18.140 | 4.8863 | 4.8984 | 0.180 | 1043 | 139 | 14.3 | 13.14 |
| 18.650 | 4.7538 | 4.7656 | 0.120 | 1063 | 142 | 14.5 | 0.91 |
| 19.140 | 4.6332 | 4.6447 | 0.140 | 7310 | 144 | 100.0 | 32.77 |
| 19.820 | 4.4757 | 4.4869 | 0.160 | 3624 | 146 | 49.6 | 9.02 |
| 20.080 | 4.4184 | 4.4294 | 0.140 | 5402 | 149 | 73.9 | 21.06 |
| 20.385 | 4.3530 | 4.3638 | 0.160 | 2652 | 149 | 36.3 | 23.25 |
| 21.215 | 4.1845 | 4.1949 | 0.160 | 369 | 154 | 5.0 | 5.78 |
| 21.890 | 4.0570 | 4.0670 | 0.200 | 773 | 156 | 10.6 | 3.09 |
| 22.630 | 3.9259 | 3.9357 | 0.280 | 4277 | 161 | 58.5 | 74.66 |
| 23.210 | 3.8291 | 3.8386 | 0.120 | 484 | 164 | 6.6 | 3.33 |
| 24.355 | 3.6516 | 3.6607 | 0.060 | 2725 | 169 | 37.3 | 1.16 |
| 24.610 | 3.6144 | 3.6234 | 0.140 | 3540 | 172 | 48.4 | 17.08 |
| 24.995 | 3.5596 | 3.5684 | 0.100 | 529 | 174 | 7.2 | 1.01 |
| 25.260 | 3.5228 | 3.5316 | 0.120 | 557 | 174 | 7.6 | 3.02 |
| 26.575 | 3.3514 | 3.3597 | 0.240 | 2421 | 182 | 33.1 | 42.58 |
| 27.155 | 3.2811 | 3.2893 | 0.140 | 676 | 185 | 9.2 | 1.32 |
| 27.310 | 3.2629 | 3.2710 | 0.100 | 767 | 185 | 10.5 | 2.75 |
| 27.865 | 3.1991 | 3.2071 | 0.120 | 420 | 188 | 5.7 | 1.08 |
| 28.210 | 3.1608 | 3.1686 | 0.100 | 1467 | 190 | 20.1 | 0.79 |
| 28.325 | 3.1482 | 3.1560 | 0.140 | 1789 | 190 | 24.5 | 4.41 |
| 28.650 | 3.1132 | 3.1210 | 0.180 | 1204 | 190 | 16.5 | 11.65 |
| 29.520 | 3.0234 | 3.0309 | 0.220 | 1011 | 196 | 13.8 | 15.74 |
| 30.250 | 2.9521 | 2.9594 | 0.120 | 159 | 199 | 2.2 | 1.22 |
| 31.105 | 2.8729 | 2.8800 | 0.360 | 282 | 204 | 3.9 | 8.14 |
| 31.905 | 2.8026 | 2.8096 | 0.100 | 339 | 207 | 4.6 | 0.96 |
| 32.350 | 2.7651 | 2.7720 | 0.120 | 237 | 210 | 3.2 | 3.01 |
| 33.300 | 2.6884 | 2.6950 | 0.180 | 1347 | 216 | 18.4 | 14.06 |
| 33.640 | 2.6620 | 2.6686 | 0.100 | 404 | 216 | 5.5 | 1.45 |
| 34.880 | 2.5701 | 2.5765 | 0.200 | 202 | 222 | 2.8 | 1.04 |
| 35.275 | 2.5422 | 2.5486 | 0.240 | 299 | 225 | 4.1 | 4.84 |
| 36.055 | 2.4890 | 2.4952 | 0.280 | 202 | 228 | 2.8 | 3.78 |
| 36.910 | 2.4333 | 2.4393 | 0.320 | 169 | 234 | 2.3 | 0.90 |
| 37.160 | 2.4175 | 2.4235 | 0.120 | 216 | 234 | 3.0 | 2.14 |
| 37.680 | 2.3853 | 2.3912 | 0.240 | 240 | 237 | 3.3 | 1.58 |
| 39.435 | 2.2831 | 2.2888 | 0.280 | 449 | 246 | 6.1 | 2.67 |
| 39.675 | 2.2698 | 2.2755 | 0.080 | 396 | 246 | 5.4 | 0.82 |
| 40.325 | 2.2347 | 2.2403 | 0.160 | 520 | 250 | 7.1 | 0.95 |
| 40.930 | 2.2031 | 2.2086 | 0.120 | 480 | 253 | 6.6 | 2.66 |
| 41.445 | 2.1769 | 2.1823 | 0.240 | 372 | 256 | 5.1 | 2.65 |
| 41.990 | 2.1499 | 2.1552 | 0.120 | 538 | 259 | 7.4 | 1.31 |
| 42.670 | 2.1172 | 2.1225 | 0.160 | 428 | 262 | 5.9 | 1.45 |
| 43.145 | 2.0950 | 2.1002 | 0.120 | 433 | 266 | 5.9 | 1.50 |
| 44.190 | 2.0478 | 2.0529 | 0.160 | 376 | 269 | 5.1 | 0.89 |
| 46.095 | 1.9675 | 1.9724 | 0.160 | 279 | 279 | 3.8 | 0.86 |
| 46.510 | 1.9509 | 1.9558 | 0.240 | 310 | 282 | 4.2 | 0.87 |
| 48.305 | 1.8826 | 1.8872 | 0.200 | 506 | 292 | 6.9 | 2.06 |
| 48.900 | 1.8610 | 1.8657 | 0.240 | 615 | 296 | 8.4 | 1.67 |
| 50.330 | 1.8115 | 1.8160 | 0.160 | 437 | 303 | 6.0 | 1.73 |
| 51.035 | 1.7881 | 1.7925 | 0.080 | 416 | 306 | 5.7 | 0.93 |
| 53.550 | 1.7099 | 1.7141 | 0.480 | 177 | 317 | 2.4 | 2.84 |
| 54.500 | 1.6823 | 1.6865 | 0.400 | 130 | 324 | 1.8 | 1.37 |
| 55.420 | 1.6565 | 1.6606 | 0.320 | 130 | 328 | 1.8 | 1.72 |
| 56.220 | 1.6348 | 1.6389 | 0.320 | 121 | 331 | 1.7 | 0.87 |
| 56.770 | 1.6203 | 1.6243 | 0.240 | 142 | 335 | 1.9 | 1.59 |
| 57.405 | 1.6039 | 1.6079 | 0.240 | 112 | 339 | 1.5 | 1.19 |
| 58.500 | 1.5764 | 1.5804 | 0.240 | 67 | 342 | 0.9 | 1.57 |

In the light of the pharmaceutical efficacy of flibanserin, the present invention furthermore relates to the use of flibanserin polymorph A as a medicament.

A further aspect of the present invention relates to the use of flibanserin polymorph A for preparing a pharmaceutical composition for treating diseases in which the use of compounds displaying affinity for the 5-HT_{1A} and 5-HT₂-receptor may have a therapeutic benefit.

A further aspect of the present invention relates to the use of flibanserin polymorph A for preparing a pharmaceutical composition for treating a disease seleceted from depression, schizophrenia, Parkinson, anxiety, sleep disturbances, sexual and mental disorders and age associated memory impairment.

In particular, the instant invention relates to the use of flibanserin polymorph A for the preparation of a medicament for the treatment of disorders of sexual desire. In a preferred embodiment the invention relates to the use of flibanserin polymorph A for the preparation of a medicament for the treatment of disorders selected from the group consisting of Hypoactive Sexual Desire Disorder, loss of sexual desire, lack of sexual desire, decreased sexual desire, inhibited sexual desire, loss of libido, libido disturbance, and frigidity.

Particular preferred according to the invention is the use of flibanserin polymorph A for the preparation of a medicament for the treatment of disorders selected from the group consiting of Hypoactive Sexual Desire Disorder, loss of sexual desire, lack of sexual desire, decreased sexual desire, inhibited sexual desire.
In a particularity preferred embodiment the invention relates to the use of flibanserin polymorph A for the preparation of a medicament for the treatment of disorders selected from the group of Hypoactive Sexual Desire Disorder and loss of sexual desire.

The aforementioned therapeutic effects of flibanserin polymorph A can be achieved in men and women. However, according to a further aspect of the invention the use of flibanserin polymorph A for the preparation of a medicament for the treatment of female sexual dysfunction is preferred.

The beneficial effects of flibanserin polymorph A can be observed regardless of whether the disturbance existed lifelong or was acquired, and independent of etiologic origin (organic - both, physically and drug induced-, psychogen, a combination of organic - both, physically and drug induced-, and psychogen, or unknown).

As a further feature of the present invention there are provided pharmaceutical compositions comprising as an active ingredient flibanserin polymorph A in addition with one or more pharmaceutical carrier, diluents or excipients. For pharmaceutical administration flibanserin polymorph A may be incorporated into the conventional pharmaceutical preparation in solid, liquid or spray form. The composition may, for example, be presented in a form suitable for oral, rectal, parenteral administration or for nasal inhalation: preferred forms includes for example, capsules, tablets, coated tablets, ampoules, suppositories and nasal spray.
The active ingredient may be incorporated in excipients or carriers conventionally used in pharmaceutical compositions such as, for example, talc, arabic gum, lactose, gelatine, magnesium stearate, com starch, acqueous or non acqueous vehicles, polyvynil pyrrolidone, semisynthetic glicerides of fatty acids, benzalconium chloride, sodium phosphate , EDTA, polysorbate 80. The compositions are advantageously formulated in dosage units, each dosage unit being adapted to supply a single dose of the active ingredient. Each dosage unit may conveniently contain from 0,01 mg to 100 mg, preferably from 0,1 to 50 mg.

## Claims

1. Crystalline polymorph A (form A) of flibanserin **1**, 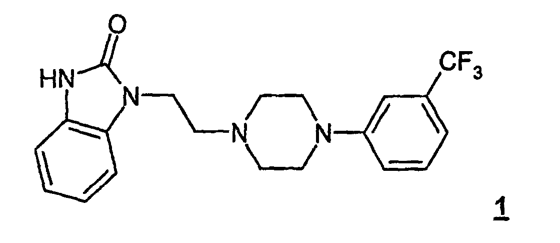 having an endothermic maximum at 161°C which occurs during thermal analysis using DSC.

2. Flibanserin **1** comprising form A according to claim 1.

3. Process for the technical preparation of flibanserin **1** according to claim 1 or 2, 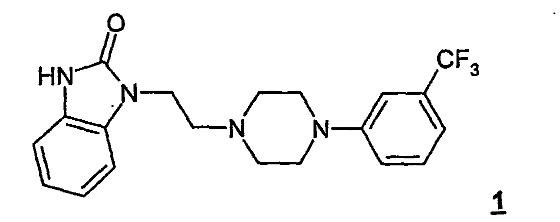 **characterized in that** in a first reaction step a benzimidazolone 2 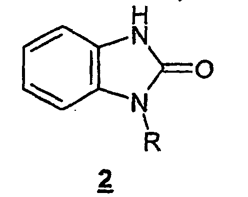 wherein R denotes a suitable amino protecting group is reacted with a piperazine **3** 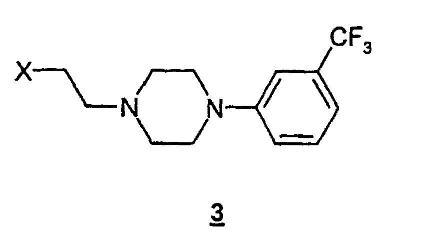 wherein X is a leaving group selected from chlorine, bromine, iodine, methanesulphonate, trifluoromethanesulphonate and para-toluenesulphonate. in a suitable solvent selected from water, alcohols, mixtures of water with alcohols, polar aprotic solvents and mixtures thereof with water in presence of a suitable base and that in a second reaction step the amino protecting group R is cleaved under suitable cleaving conditions.

4. Process according to claim 3, charcterized in that the reaction of **2** with **3** is conducted at a temperature of at least 50°C.

5. Process according to claim 4, **characterized in that** heating is conducted for about 10 minutes to about 12 hours.

6. Polymorph A of flibanserin **1** obtainable according to one of claims 3 to 5.

7. Polymorph A of flibanserin **1** according to one of claims 1, 2 or 6 for use as a medicament.

8. Use of polymorph A of flibanserin **1** according to one of claims 1, 2 or 6 for preparing a medicament for treating diseases in which the use of therapeutic effective amounts of compounds displaying affinity for the 5-HT_{1A} and 5-HT₂-receptor display a therapeutic benefit.

9. Use of polymorph A of flibanserin **1** according to one of claims 1, 2 or 6 for preparing a medicament for treating a disease seleceted from depression, schizophrenia, Parkinson, anxiety, sleep disturbances, sexual and mental disorders and age associated memory impairment.

10. Pharmaceutical compositions comprising as an active ingredient polymorph A of flibanserin **1** according to one of claims 1, 2 or 6 optionally in admixture with one or more pharmaceutical carriers, diluents or excipients.

## Patentansprüche

1. Kristallines Polymorph A (Form A) von Flibanserin **1** 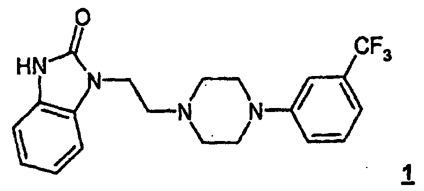 mit einem endothermen Maximum bei 161°C, das während der Thermoanalyse unter Anwendung der DSK auftritt.

2. Flibanserin **1**, umfassend die Form A nach Anspruch 1.

3. Verfahren zur technischen Herstellung von Flibanserin **1** nach Anspruch 1 oder 2, 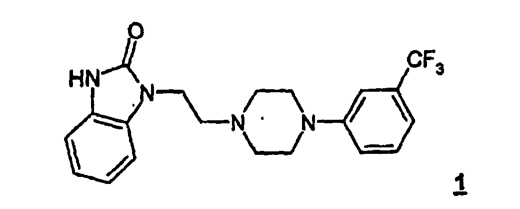 **dadurch gekennzeichnet, dass** in einem ersten Reaktionsschritt ein Benzimidazolon **2** 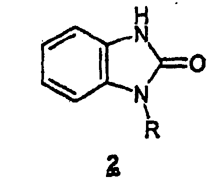 worin R eine geeignete Aminoschutzgruppe darstellt, mit einem Piperazin **3** 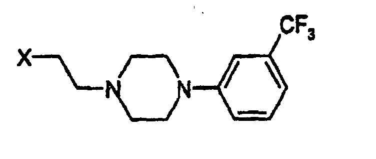 worin X eine Abgangsgruppe, ausgewählt unter Chlor, Brom, lod, Methansulfonat, Trifluormethansulfonat und p-Toluolsulfonat ist,
in einem geeigneten Lösungsmittel, ausgewählt aus Wasser, Alkoholen, Mischungen von Wasser mit Alkoholen, polaren aprotischen Lösungsmitteln und Mischungen davon mit Wasser, in Gegenwart einer geeigneten Base umgesetzt wird und dass in einem zweiten Reaktionsschritt die Aminoschutzgruppe R unter geeigneten Spaltungsbedingungen abgespalten wird.

4. Verfahren nach Anspruch 3, **dadurch gekennzeichnet, dass** die Reaktion von **2** mit **3** bei einer Temperatur von mindestens 50°C durchgeführt wird.

5. Verfahren nach Anspruch 4, **dadurch gekennzeichnet, dass** das Erhitzen etwa 10 Minuten bis etwa 12 Stunden durchgeführt wird.

6. Polymorph A von Flibanserin 1, erhältlich gemäß einem der Ansprüche 3 bis 5.

7. Polymorph A von Flibanserin **1** nach einem der Ansprüche 1, 2 oder 6 zur Verwendung als Arzneimittel.

8. Verwendung des Polymorph A von Flibanserin **1** nach einem der Ansprüche 1, 2 oder 6 zur Herstellung eines Arzneimittels für die Behandlung von Krankheiten, in denen die Anwendung von therapeutisch wirksamen Mengen von Verbindungen, die eine Affinität für den 5-HT_{1A}- und 5HT₂-Rezeptor zeigen, von therapeutischem Nutzen ist.

9. Verwendung des Polymorph A von Flibanserin **1** nach einem der Ansprüche 1, 2 oder 6 zur Herstellung eines Arzneimittels für die Behandlung einer Krankheit, ausgewählt aus Depression, Schizophrenie, Parkinson, Angst, Schlafstörungen, sexuellen und mentalen Störungen und altersbedingtem Gedächtnisschwund.

10. Pharmazeutische Zusammensetzungen, umfassend als Wirkstoff Polymorph A von Flibanserin **1** nach einem der Ansprüche 1, 2 oder 6, gegebenenfalls in Mischung mit einem oder mehreren pharmazeutischen Trägern, Verdünnungsmitteln oder Excipienten.

## Revendications

1. Polymorphe cristallin A (forme A) de flibansérine 1, 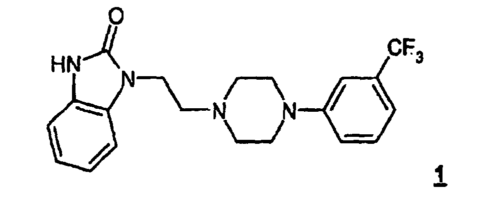 ayant un maximum endothermique à 161°C qui apparaît lors de l'analyse thermique par calorimétrie à balayage différentiel (DSC).

2. Flibansérine 1 comprenant la forme A selon la revendication 1.

3. Processus de préparation technique de flibansérine 1 selon la revendication 1 ou 2, 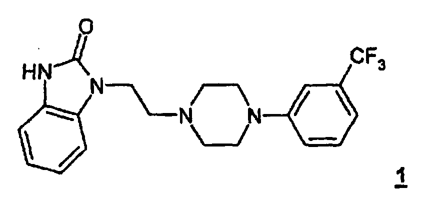 **caractérisé en ce qu'**au cours d'une première étape de réaction un benzimidazolone 2 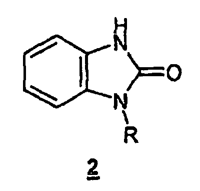 dans lequel R représente un groupe de protection amino adapté, réagit avec une pipérazine 3 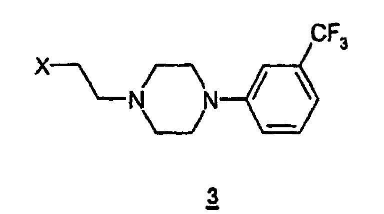 dans laquelle X est un groupe partant choisi parmi le chlore, le brome, l'iode, le méthanesulfonate, le trifluorométhanesulfonate, et le para-toluènesulfonate, dans un solvant adapté choisi parmi l'eau, les alcools, les mélanges d'eau et d'alcools, les solvants polaires aprotiques et les mélanges de ceux-ci avec de l'eau en présence d'une base adaptée et **en ce qu'**au cours d'une seconde étape de réaction, le groupe de protection amino R est clivé dans des conditions de clivage adaptées.

4. Processus selon la revendication 3, **caractérisé en ce que** la réaction de 2 avec 3 est réalisée à une température d'au moins 50°C.

5. Processus selon la revendication 4, **caractérisé en ce que** le chauffage est réalisé pendant environ 10 minutes à environ 12 heures.

6. Polymorphe A de flibansérine 1 pouvant être obtenu selon l'une des revendications 3 à 5.

7. Polymorphe A de flibansérine 1 selon l'une des revendications 1, 2 ou 6, destiné à être utilisé en tant qu'un médicament.

8. Utilisation du polymorphe A de flibansérine 1 selon l'une des revendications 1, 2 ou 6, pour la préparation d'un médicament destiné à traiter des maladies dans lesquelles l'utilisation de quantités thérapeutiques efficaces de composés présentant une affinité pour les récepteurs 5-HT_{1A} et 5-HT₂ présente un bénéfice thérapeutique.

9. Utilisation du polymorphe A de flibansérine 1 selon l'une quelconque des revendications 1, 2 ou 6, pour la préparation d'un médicament destiné à traiter une maladie choisie parmi la dépression, la schizophrénie, la maladie de Parkinson, l'anxiété, les troubles du sommeil, les troubles mentaux et sexuels et l'altération de la mémoire liée à l'âge.

10. Compositions pharmaceutiques comprenant en tant qu'un agent actif le polymorphe A de flibansérine 1 selon l'une quelconque des revendications 1, 2 ou 6, facultativement mélangé avec un ou plusieurs vecteurs, diluants ou excipients pharmaceutiques.
